# NEUE EUROPÄISCHE PATENTSCHRIFT

(11) **EP 0 743 948 B2**
(45) Veröffentlichungstag und Bekanntmachung des Hinweises auf die Entscheidung über den Einspruch: **08.10.2003**
(45) Hinweis auf die Patenterteilung: 07.07.1999
(21) Anmeldenummer: 95907640.7
(22) Anmeldetag: 03.02.1995
(51) Int. Cl.: C07H 1/08, C07H 21/00, C12N 15/10, C12P 19/34

(54) **CHROMATOGRAPHISCHE ISOLIERUNG VON NUCLEINSÄUREN**
SEPARATION OF NUCLEIC ACIDS BY CHROMATOGRAPHY
SEPARATION DES ACIDES NUCLEIQUES PAR CHROMATOGRAPHIE

(30) Priorität: 07.02.1994 DE 4403692; 07.02.1994 DE 4403693; 01.09.1994 DE 4431125; 14.09.1994 DE 4432654
(43) Veröffentlichungstag der Anmeldung: 27.11.1996
(73) Patentinhaber: QIAGEN GmbH, 40724 Hilden (DE)
(72) Erfinder: COLPAN, Metin, D-45219 Essen (DE); SCHORR, Joachim, D-40231 Düsseldorf (DE)
(74) Vertreter: Meyers, Hans-Wilhelm, Dr.Dipl.-Chem.
(86) Internationale Anmeldenummer: EP9500390
(87) Internationale Veröffentlichungsnummer: WO95021178

(56) Entgegenhaltungen:
- EP-A- 0 512 767
- EP-A- 0 512 768
- WO-A-93/11221
- DE-A- 4 321 904
- US-A- 4 935 342
- Oxford Dictionary of Biochem. and Mol.Biol. (1997)
- FPLC TM lon Exchange and Chromatofocusing - Principles and Methods, Pharmacia Laboratory Separation Division, Sweden, 1985
- English language translation of EP-B-0743948
- Oiagen Plasmid Handbook, Spring 1992
- High-performance Liquid Chromatography and Lipids; A Practical Guide 1987
- Römpp, Lexikon Biochemie & Molekularbiologie Thieme (2000)

## Beschreibung

Gegenstand der vorliegenden Erfindung ist ein Verfahren zur chromatographischen Isolierung von Nucleinsäuren, die Verwendung von Isopropanol in wäßrigen Lösungen zur Steigerung der Transfektionseffizienz von Nucleinsäuren in pro- und eukaryontischen Zellen sowie eine wäßrige Isopropanollösung.

Die DE-A-36 39 949 der Anmelderin betrifft ein Verfahren zur Isolierung und Reinigung von Nucleinsäuren, insbesondere langkettiger Nucleinsäuren. Dabei werden wäßrige Lösungen (Puffer) relativ niedriger lonenstärke verwendet, um die zunächst bei niedriger lonenstärke an einer Anionenaustauschermatrix adsorbierten Nucleinsäure zu waschen. Danach werden die Nucleinsäuren mit Puffern höherer lonenstärke von der Matrix desorbiert. Ethanol kann dabei unter anderem zur Präzipitation von Nucleinsäuren, insbesondere DNA, eingesetzt werden. Niedere Alkohole enthaltende Lösungen werden in der DE-A-43 21 904 der Anmelderin vorgeschlagen. Hierbei werden die niedere aliphatische Alkohole enthaltenden Lösungen in Zusammenwirkung mit chaotropen lonen hoher Konzentration verwendet, um Nucleinsäuren an nicht mit Anionenaustauschergruppen modifizierten anorganischen Materialien zu adsorbieren.

Die so isolierte Nucleinsäure wird anschließend häufig durch chemische oder physikalische Methoden in eukaryontische oder prokaryontische Zellen eingeschleust. Dieses als Transfektion bezeichnete Verfahren wird angewendet, um den regulatorischen Einfluß von bestimmten Genen bzw. deren Expressionsprodukte auf das Genom der Wirtszelle zu studieren. Solche Verfahren sind sehr arbeits- und zeitaufwendig, da regelmäßig 10 bis 14 Tage für die Durchführung und Auswertung eines einzelnen Experimentes eingeplant werden müssen. Daher ist man sehr daran interessiert, daß beispielsweise die zu untersuchende Plasmid-DNA in möglichst viele Zellen einer Zellkultur eingeschleust wird. Die als Transfektionseffizienz bezeichnete Frequenz der Aufnahme angebotener Fremd-DNA ist von verschiedenen Faktoren abhängig. Neben der Qualität der Zellkultur und Art der Transfektionsmethode, hängt die Transfektionseffizienz üblicherweise ganz entscheidend von der Qualität bzw. Reinheit der verwendeten Plasmid-DNA ab.

In Ehlert et al., Biotechniques 14, 1993, 546, konnte gezeigt werden, daß Plasmid-DNA, die unter Verwendung des QIAGEN® Anionenaustauschermaterials gemäß des in DE-A-36 39 949 beschriebenen Verfahrens präpariert wurde, eine bis zu 70% bessere Transfektionseffizienz ergibt, als die über Caesiumchlorid-Dichtegradientenzentrifugation gereinigte DNA.

Durch die Verwendung von Isopropanol in wäßrigen Lösungen, die zur Isolierung von Nucleinsäuren eingesetzt werden, konnte überraschenderweise die aus Biotechniques 14, 1993, 546 bekannte Transfektionsrate um bis zu 20% gesteigert werden.

Die Erfindung betrifft somit eine gegenüber der aus der DE-A-36 39 949 bekannten Isolierung eine Isolierung von Nucleinsäuren, die eine bessere Transfektionsrate aufweist, durch die Verwendung von isopropanolhaltigen wäßrigen Lösungen zur Trennung und Isolierung der Nucleinsäuren an Anionenaustauschern.

In einer bevorzugten Ausführungsform des erfindungsgemäßen Verfahrens wird dabei das Isopropanol in einer Menge von 1 - 50 Vol.-%, insbesondere 5 - 25 Vol.-%, besonders bevorzugt von 10 bis 15 Vol.-%, in den wäßrigen Lösungen vorliegen. Dabei können die wäßrigen Lösungen insbesondere auch für Anionenaustauscher übliche chromatographische Salze mono- oder divalenter Kationen, wie Natriumchlorid, Kaliumchlorid, etc. oder Kombinationen davon, enthatten. Die wäßrigen Lösungen sind vorzugsweise mit in der Molekularbiologie gebräuchlichen Puffersubstanzen, wie TRIS/HCl, MOPS, etc., gepuffert und weisen pH-Werte zwischen 6 und 9 auf.

Das erfindungsgemäße Verfahren mittels erfindungsgemäßer Verwendung des Isopropanols läßt sich insbesondere bei der Isolation von DNA, wie Plasmid-DNA, anwenden, ist jedoch nicht auf diese DNA-Arten beschränkt.

Die unter erfindungsgemäßer Verwendung von Isopropanol mittels des erfindungsgemäßen Verfahrens erhaltenen Nucleinsäuren sind insbesondere für den Einsatz in gentherapeutischen Verfahren geeignet.

Erfindungsgemäß werden ebenfalls Lösungen beansprucht, die Isopropanol in Mengen von 5 bis 60 Vol.-% enthalten können. Dies sind insbesondere die Waschpuffer, die gemäß DE 36 39 949 A1 eingesetzt werden können. Diese Puffer enthalten 0,5 bis 1,5 M Natriumchlorid oder Kaliumchlorid, 20 bis 80 mM MOPS oder TRIS/HCl bei einem pH-Wert von 6 bis 8. Erfindungsgemäß ebenfalls beansprucht werden wäßrige Lösungen unter Verwendung von 5 bis 60 Vol.-% Isopropanol enthaltend darüber hinaus 1,0 bis 2,0 M Natriumchlorid oder Kaliumchlorid, 20 bis 80 mM TRIS/ HCI bei einem pH-Wert von 8 bis 9.

Die DE-A-39 13 814 beschreibt in allgemeiner Form wäßrige Systeme in Puffern, die zur Elektroelution von Gelen verwendet werden können. Die dort beschriebenen Puffersysteme weisen beispielsweise Natriumchlorid, Puffersalze, wie MOPS, und niedere Alkohole, insbesondere C1 bis C4-Alkohole, auf. Die Salzkonzentration dieser Puffer ist jedoch recht hoch, da ausdrücklich 1,5 M Natriumchlorid oder mehr verlangt werden.

In bevorzugter Weise kann ein isopropanolhaltiges wäßriges System bei dem Verfahren zur Abreicherung oder Entfernung von Endotoxinen, wie es in der DE-A-44 31 125 vorgeschlagen wird, eingesetzt werden. Diese Patentanmeldung schlägt ein Verfahren vor, bei dem Endotoxine aus für den therapeutischen Einsatz vorgesehenen Wirkstoff enthaltenden Präparaten abgereichert oder entfernt werden. Die Präparate werden dabei vorzugsweise genund/oder biotechnologisch aus natürlichen Quellen gewonnen. Die Abreicherung oder Entfernung der Endotoxine erfolgt durch Behandlung der Proben mit chromatographischem Material, wobei die natürlichen Quellen aufgeschlossen werden, die erhaltenen Fraktionen gegebenenfalls zentrifugiert, filtriert oder mit affinitätschromatographischem Material behandelt werden. Die erhaltenen Fraktionen werden mit einer wäßrigen Salzlösung und Detergenzien vorinkubiert, mit Anionenaustauschermaterial behandelt und danach mit einer weiteren Salzlösung gewaschen. Die Wirkstoffe werden von dem Anionenaustauscher eluiert, um dann in an sich bekannter Weise weiter aufgereinigt zu werden. Wird beispielsweise Nucleinsäure isoliert, die in gentherapeutischen Verfahren eingesetzt werden kann und einer Endotoxinentfernung oder Abreicherung gemäß DE-A-44 31 125 unterzogen wurde, so kann eine Steigerung der Transfektionseffizienz bis zu 50% beobachtet werden im Vergleich zu Präparationen, in denen Ethanol in den Präparationslösungen verwendet wurde.

Die Erfindung wird anhand des folgenden Beispiels näher erläutert.

### Beispiel 1

Als Nucleinsäuren wurden im folgenden Plasmid-DNA aus E. coli analog dem in DE-A-36 39 949 genannten Verfahren getrennt und isoliert. Dabei wird die Nucleinsäure an dem in der DE 36 39 949 A1 beschriebenen Anionenaustauschermaterial (QIAGEN®, Diagen GmbH, Deutschland) adsorbiert. Es wurde mit einem Puffer der Zusammensetzung 1,0 M NaCl, 50 mM MOPS, 15% Isopropanol, pH 7,0 gewaschen. Danach wurde die Plasmid-DNA mit dem isopropanolhaltigen Puffer der Zusammensetzung 1,25 M NaCl, 50 mM TRIS/HCl, 15 Vol.-% Isopropanol, pH 8,5 eluiert.

Dabei zeigte sich, daß die Ausbeute von Plasmid-DNA aus E. coli gegenüber der bekannten Präparation unter Verwendung von Ethanol um ca. 10% gesteigert werden konnte.

Mit der so erhaltenen DNA wurden dann Transfektionsexperimente durchgeführt. Die Transfektionseffizienz wurde über die Messung der lacZ-Aktivität bestimmt, wobei NIH 3T3-Zellen verwendet wurden. Diese Zellen wurden mit Hilfe der Kalziumphosphatmethode (Graham, F. L. and A. J. van der Eb (1973) "A new technique for the assay of infectivity of human adenovirus 5 DNA", Virology 52: 456- 467) mit 1 µg des Reporterkonstrukts pRSVlacZ transfiziert (Lucibello, F. c. and R. Müller (1989) "Sensitive microscale assay for the analysis of promotor activity in eukaryotic cells", Methods Mol. Biol. 1:9 - 18).

Die Tabelle zeigt die Transfektionseffizienz bei Verwendung verschiedener Alkohole, Ethanol reinst und vergällt, Isopropanol und n-Butanol.

| | % lacZ-Aktivität | | | |
|---|---|---|---|---|
| Experiment No. | 15% Ethanol reinst in Puffer QBT, QC, QF | 15% Ethanol vergällt in Puffer QBT, QC, QF | 15% Isopropanol in Puffer QBT, QC, QF | 15% n-Butanol in Puffer QBT, QC, QF |
| 1 | 80 | 78 | 95 | 20 |
| 2 | 75 | 70 | 92 | 12 |
| 3 | 70 | 70 | 98 | 9 |
| 4 | 85 | 80 | 100 | 15 |
| 5 | 83 | 78 | 97 | 18 |

Deutlich erkennbar wird aus den Angaben der Tabelle die überraschend hohe Transfektionseffizienz von DNA, die mit isopropanolhaltigen Puffern gewonnen wurden.

### Beispiel 2

Plasmid-DNA wurde gemäß Beispiel 1 isoliert. Das Lysat wurde jedoch vor dem Auftragen auf die Anionenaustauschersäule über eine lose Schüttung von Diatomeenerde, wie in der DE-A-44 32 654 beschrieben, gegeben. Anschließend wurde eine Endotoxinabreicherung gemäß dem in DE-A-44 31 125 beschriebenen Verfahren durchgeführt. Dabei wurde eine wäßrige Lösung verwendet, die 750 mM Natriumchlorid, 10% Triton-X-100/50 mM MOPS beinhaltete bei einem pH von 7,0, eingesetzt. Die so präparierte DNA wurde mit Hilfe von kationischen Liposomen (DOTAP, Boehringer Mannheim) in LMH-Leberzellen transfiziert. Dabei zeigt sich eine 50%ige Steigerung der Transfektionseffizienz verglichen mit einer DNA-Präparation gemäß dem Stand der Technik aus DE-A-36 39 664.

## Patentansprüche

1. Verfahren zur chromatographischen Isolierung von Nucleinsäuren an Anionenaustauschern unter Verwendung von Isopropanol in wäßriger Lösung als mobile Phase.

2. Verwendung von Isopropanol in wäßrigen Lösungen für die chromatographische Isolierung von Nucleinsäuren an Anionenaustauschern zur Steigerung der Transfektionseffizienz der isolierten Nucleinsäuren in pro- und eukaryontischen Zellen.

3. Verwendung nach Anspruch 2, wobei das Isopropanol in Mengen von 1 - 50 Vol.-% in den wäßrigen Lösungen vorliegt.

4. Verwendung nach Anspruch 2, wobei das Isopropanol in Mengen von 5 - 25 Vol.-% in den wäßrigen Lösungen vorliegt.

5. Verwendung nach Anspruch 2, wobei das Isopropanol in Mengen von 10 - 15 Vol.-% in den wäßrigen Lösungen vorliegt.

6. Verwendung nach mindestens einem der Ansprüche 2 bis 5, wobei die wäßrige Lösung für die Anionenaustauscher-Chromatographie übliche Salze mono- oder divalenter Kationen, wie Natriumchlorid, Kalziumchlorid oder Kaliumchlorid enthält.

7. Verwendung nach mindestens einem der Ansprüche 2 bis 6, wobei die wäßrige Lösung eine gepufferte Lösung ist.

8. Verwendung nach mindestens einem der Ansprüche 2 bis 7, wobei die Nucleinsäure-DNA Plasmid-DNA ist.

9. Verwendung nach mindestens einem der Ansprüche 2 bis 8 zur Herstellung von Nucleinsäuren für die Gentherapie.

10. Wäßrige Lösung enthaltend 0,5 bis 1,5 M Natriumchlorid oder Kaliumchlorid, 10 bis 100 mM MOPS oder TRIS/HCl sowie 5 bis 60 Vol.-% Isopropanol bei einem pH-Wert von 6 bis 8.

11. Wäßrige Lösung enthaltend 1,0 bis 2,0 M Natriumchlorid oder Kaliumchlorid, 10 bis 100 mM TRIS/HCl, 5 bis 60 Vol.-% Isopropanol bei einem pH-Wert von 8 bis 9.

## Claims

1. A method for the chromatographic isolation of nucleic acids on anion-exchangers using isopropanol in aqueous solution as the mobile phase.

2. Use of isopropanol in aqueous solutions for the chromatographic isolation of nucleic acids on anion-exchangers for enhancing the transfection efficiency of the isolated nucleic acids in prokaryotic and eukaryotic cells.

3. The use according to claim 2, wherein said isopropanol is present in said aqueous solutions in amounts of from 1 to 50% by volume.

4. The use according to claim 2, wherein said isopropanol is present in said aqueous solutions in amounts of from 5 to 25% by volume.

5. The use according to claim 2, wherein said isopropanol is present in said aqueous solutions in amounts of from 10 to 15% by volume.

6. The use according to at least one of claims 2 to 5, wherein said aqueous solution contains salts of monovalent or divalent cations commonly used for anion exchange chromatography, such as sodium chloride, calcium chloride or potassium chloride.

7. The use according to at least one of claims 2 to 6, wherein said aqueous solution is a buffered solution.

8. The use according to at least one of claims 2 to 7, wherein the nucleic acid DNA is plasmid DNA.

9. The use according to at least one of claims 2 to 8 for the preparation of nucleic acids for gene therapy.

10. An aqueous solution containing 0.5 to 1.5 M sodium chloride or potassium chloride, 10 to 100 mM MOPS or TRIS/HCl, and from 5 to 60% by volume of isopropanol at a pH value of 6 to 8.

11. An aqueous solution containing 1.0 to 2.0 M sodium chloride or potassium chloride, 10 to 100 mM TRIS/HCl, and from 5 to 60% by volume of isopropanol at a pH value of 8 to 9.

## Revendications

1. Procédé d'isolement chromatographique d'acides nucléiques sur des échangeurs d'anions, par utilisation dlisopropanol en solution aqueuse en tant que phase mobile.

2. Utilisation dlisopropanol en solutions aqueuses pour l'isolement chromatographique d'acides nucléiques sur des échangeurs d'anions, pour augmenter le rendement de transfection des acides nucléiques isolés dans des cellules procaryotes et eucaryotes.

3. Utilisation selon la revendication 2, dans laquelle Ilisopropanal est présent dans les solutions aqueuses en des quantités de 1 à 50 % en volume.

4. Utilisation selon la revendication 2, dans laquelle Ilisopropanol est présent dans les solutions aqueuses en des quantités de 5 à 25 % en volume.

5. Utilisation selon la revendication 2, dans laquelle llisopropanol est présent dans les solutions aqueuses en des quantités de 10 à 15 % en volume.

6. Utilisation selon au moins l'une des revendications 2 à 5, dans laquelle la solution aqueuse pour la chromatographie sur échangeurs d'anions contient des sels usuels de cations mono- ou divalents, tels que le chlorure de sodium, le chlorure de calcium ou le chlorure de potassium.

7. Utilisation selon au moins l'une des revendications 2 à 6, dans laquelle la solution aqueuse est une solution tamponnée.

8. Utilisation selon au moins l'une des revendications 2 à 7, dans laquelle l'acide nucléique ADN est un ADN plasmidique.

9. Utilisation selon au moins l'une des revendications 2 à 8, pour préparer des acides nucléiques destinés à la thérapie génique.

10. Solution aqueuse contenant de 0,5 à 1,5 M de chlorure de sodium ou de chlorure de potassium, de 10 à 100 mM de MOPS ou de TRIS/HCl, ainsi que de 5 à 60 % en volume dlisopropanol, pour un pH de 6 à 8.

11. Solution aqueuse contenant de 1,0 à 2,0 M de chlorure de sodium ou de chlorure de potassium, de 10 à 100 mM de TRIS/HCl, de 5 à 60 % en volume dlisopropanol pour un pH de 8 à 9.
